# EUROPÄISCHE PATENTANMELDUNG

(11) **EP 3 907 006 A1**
(43) Veröffentlichungstag der Anmeldung: **10.11.2021**
(21) Anmeldenummer: 20173100.7
(22) Anmeldetag: 06.05.2020
(51) Int. Cl.: B01L 3/00, F16K 99/00

(54) **MIKROFLUIDISCHES VENTIL**

(71) Anmelder: Kilobaser GmbH, 8020 Graz (AT)
(72) Erfinder: Murer, Alexander, 8020 Graz (AT); Tittelbach, Bernhard, 8010 Graz (AT); Jost, Martin, 8010 Graz (AT); Diethardt, Reinhard, 8741 Weißkirchen (AT)
(74) Vertreter: KLIMENT & HENHAPEL

(57) **Zusammenfassung**

Die Erfindung betrifft ein Mikrofluid-Ventil umfassend eine Trägerschicht (7) und eine auf einer Oberfläche der Trägerschicht (7) angeordnete flexible Membranschicht (8), wobei die Oberfläche der Trägerschicht (7) eine Ventilkammer (5) in Form einer Kugelkalotte aufweist und eine durch die flexible Membranschicht (8) ausgebildete Membran (4) zumindest die Ventilkammer (5) überspannt, wobei mehrere in die Ventilkammer (5) einmündende mikrofluidische Kanäle (1,2,3) in der Oberfläche der Trägerschicht (7) ausgebildet sind.

Um ein Mikrofluid-Ventil vorzuschlagen, welches die Vorteile der wirtschaftlichen Herstellung mit einer einfachen Integrierbarkeit in ein mikrofluidisches System, insbesondere in einen mikrofluidischen Chip, zu kombinieren ist erfindungsgemäß vorgesehen, dass ein Zuleitungskanal (1) und ein Ableitungskanal (3) durch einen mikrofluidischen Verbindungskanal (6) miteinander verbunden sind,
wobei der Verbindungskanal (6) und die Ventilkammer (5) derart zueinander positioniert sind,
dass im geschlossenen Zustand der Membran (4) ein Fluid aus dem Zuleitungskanal (1) über den Verbindungskanal (6) in den Ableitungskanal (3) fließen kann, um die Ventilkammer (5) zu überbrücken, während der zumindest eine Einleitungskanal (2) durch die Membran (4) verschlossen ist.

## Beschreibung

### GEBIET DER ERFINDUNG

Die Erfindung betrifft ein Mikrofluid-Ventil umfassend eine Trägerschicht und eine auf einer Oberfläche der Trägerschicht angeordnete flexible Membranschicht,
wobei die Oberfläche der Trägerschicht eine Ventilkammer in Form einer Kugelkalotte aufweist und eine durch die flexible Membranschicht ausgebildete Membran zumindest die Ventilkammer überspannt, wobei mehrere in die Ventilkammer einmündende mikrofluidische Kanäle in der Oberfläche der Trägerschicht ausgebildet sind.

### STAND DER TECHNIK

In mikrofluidischen Systemen, insbesondere in mikrofluidischen Chips, wird das an sich bekannte mikrofluidische Verhalten von Flüssigkeiten ausgenützt, wobei der lichte Durchmesser der mikrofluidischen Kanäle des Systems in der Regel weniger als einen Millimeter (1 mm) beträgt und üblicherweise zwischen 100 Nanometer (100 nm) und 500 Mikrometer (500 µm) liegt. Dabei bilden sich Strömungen mit besonders niedrigen Reynolds Zahlen aus, was einer nahezu ausschließlich laminaren Strömung entspricht. Derartige mikrofluidische Systeme werden insbesondere zur Miniaturisierung, zur Automatisierung und zur verbesserten Integration von an sich bekannten makrofluidischen Systemen eingesetzt.

Mikrofluidische Ventile werden in solchen mikrofluidischen Systemen eingesetzt, um den Durchfluss zu regulieren, insbesondere um den Durchfluss durch einen mikrofluidischen Kanal abzusperren oder freizugeben und/oder um einzelne Bereiche des Systems zumindest temporär gegenüber anderen Bereichen abzudichten.

Die Aktuierung der mikrofluidischen Ventile kann auf eine Vielzahl unterschiedlicher technischer Prinzipien beruhen, beispielsweise auf piezo-elektrischen Elementen, auf magnetischen Komponenten, auf elektrischen Komponenten oder auf Druckbeaufschlagung bzw. Änderung des Drucks.

Eine günstige Bauform eines mikrofluidischen Ventils zeichnet sich durch eine im Wesentlichen starre Trägerschicht aus, in deren Oberfläche die mikrofluidischen Kanäle, nämlich ein Zuleitungskanal und ein Ableitungskanal, ausgebildet sind, wobei auf der Oberfläche der Trägerschicht eine flexible Membranschicht aufgebracht ist. In der Oberfläche der Trägerschicht ist weiters eine Ventilkammer in Form einer Kugelkalotte vorgesehen, wobei die Kugelkalotte maximal einer Halbkugel entspricht. Sowohl der Zuleitungskanal als auch der Ableitungskanal münden in die Ventilkammer ein. Im Bereich der Ventilkammer überspannt die Membranschicht als Membran die Ventilkammer.

Wird die Membran mit Druck beaufschlagt und so in einen geschlossenen Zustand gebracht, so wird die Ventilkammer verschlossen, sodass kein Fluid vom Zuleitungskanal über die Ventilkammer in den Ableitungskanal gelangen kann. Wird der Druck auf die Membran verringert und die Membran so in einen geöffneten Zustand gebracht, so entspannt sich die Membran und nimmt ihre ursprüngliche Form ein, sodass die Ventilkammer freigegeben ist und Fluid aus dem Zuleitungskanal über die Ventilkammer in den Ableitungskanal fließen kann.

Während der einfache Aufbau eines derartigen mikrofluidischen Ventils eine wirtschaftliche Herstellung ermöglicht, lassen sich derartige mikrofluidische Ventile nur schwer oder mit großem Aufwand in komplexere mikrofluidische Systeme integrieren, in welchen verschiedene mikrofluidische Pfade durch entsprechende Ventilstellung abwechselnd freigegeben und verschlossen werden sollen. Dadurch, dass das mikrofluidische Ventil nur einen offenen und einen geschlossenen Zustand aufweist, sind entsprechende Stichkanäle zum Anschluss des Ventils an das mikrofluidische System notwendig. Insbesondere stellt sich dieses Problem bei mikrofluidschen Synthese-Chips, bei denen eine Vielzahl an unterschiedlichen Reagenzien in einer vordefinierten Reihenfolge einer Synthesekammer zugeführt werden müssen, wobei jeweils ein mikrofluidisches Ventil die Zufuhr eines Reagenz steuert.

### AUFGABE DER ERFINDUNG

Es ist daher eine Aufgabe der Erfindung die Nachteile des Stands der Technik zu überwinden und ein Mikrofluid-Ventil vorzuschlagen, welches die Vorteile der wirtschaftlichen Herstellung mit einer einfachen Integrierbarkeit in ein mikrofluidisches System, insbesondere in einen mikrofluidischen Chip, kombiniert.

### DARSTELLUNG DER ERFINDUNG

Diese Aufgabe wird in einem erfindungsgemäßen Mikrofluid-Ventil der eingangs erwähnten Art dadurch gelöst, dass die mikrofluidischen Kanäle einen Zuleitungskanal, einen Ableitungskanal und zumindest einen Einleitungskanal umfassen, wobei die mikrofluidischen Kanäle und die Membran derart ausgebildet sind, dass die Membran durch Beaufschlagung mit Druck in einen geschlossenen Zustand bringbar ist, in welchem geschlossenen Zustand die Membran in die Ventilkammer gepresst wird, um den Übertritt eines einzuleitenden Fluides aus dem zumindest einen Einleitungskanal in die Ventilkammer zu verhindern,
wobei der Zuleitungskanal und der Ableitungskanal durch einen Verbindungskanal miteinander verbunden sind,
wobei der Verbindungskanal und die Ventilkammer derart zueinander positioniert sind,
dass im geschlossenen Zustand der Membran ein zuzuführendes Fluid aus dem Zuleitungskanal über den Verbindungskanal in den Ableitungskanal fließen kann, während der zumindest eine Einleitungskanal durch die Membran verschlossen ist
und dass in einem geöffneten Zustand der Membran ein zuzuführendes Fluid aus dem Zuleitungskanal und/oder zumindest ein einzuleitendes Fluid aus dem zumindest einen Einleitungskanal in die Ventilkammer fließen können, wobei ein sich in der Ventilkammer befindliches Fluid über den Ableitungskanal aus der Ventilkammer abfließen kann.

Die erfindungsgemäße Gestaltung des Mikrofluid-Ventils zeichnet sich dadurch aus, dass zumindest drei mikrofluidische Kanäle in die Ventilkammer einmünden, nämlich sowohl der Zuleitungskanal, der Ableitungskanal und der zumindest eine Einleitungskanal. Ist die Membran im geöffneten Zustand und somit die Ventilkammer freigegeben, können Fluide je nach angelegtem Druckgefälle aus dem Zuleitungskanal und/oder aus dem zumindest einen Einleitungskanal in den Ableitungskanal fließen. Wenngleich es denkbar ist, dass aus mehreren Kanälen gleichzeitig Fluide in die Ventilkammer einfließen und gemeinsam über den Ableitungskanal abfließen, ist es bevorzugt, wenn die Druckgefälle der mikrofluidischen Kanäle im Betriebszustand so eingestellt werden, dass lediglich ein Fluidpfad durchströmt wird.

Bevorzugt ist das Mikrofluid-Ventil so ausgelegt, dass im geöffneten Zustand der Membran im Betriebszustand lediglich ein einzuleitendes Fluid aus dem jeweiligen Einleitungskanal über die Ventilkammer in den Ableitungskanal übergeht. Es ist jedoch auch denkbar, dass aus mehreren Einleitungskanälen gleichzeitig Fluide in die Ventilkammer strömen können.

Besonders bevorzugt ist genau ein Einleitungskanal vorgesehen, um einen besonders einfachen Aufbau zu erreichen, wobei sich ein derartiger Aufbau mit drei mikrofluidischen Kanälen insbesondere für die Zuleitung eines Reagenz in ein mikrofluidisches System mit mehreren seriell hintereinander geschalteten Mikrofluid-Ventilen eignet.

Als Material für die Trägerschicht eignen sich insbesondere verschiedene Kunststoffe, die vorzugsweise so ausgewählt sind, dass sie mit den durch das Mikrofluid-Ventil geführten Fluiden nicht reagieren, wobei die Trägerschicht vorzugsweise eine höhere Steifigkeit aufweist als die flexible Membranschicht. Die höhere Steifigkeit kann beispielsweise durch eine entsprechend höhere Schichtdicke der Trägerschicht im Vergleich zur Membranschicht erreicht werden, insbesondere wenn die Trägerschicht und die Membranschicht aus Kunststoff bestehen. Natürlich sind auch Glas oder glasartige Materialien als Material für die Trägerschicht denkbar.

Als Material für die flexible Membranschicht kommen ebenfalls verschiedene Kunststoffe in Frage, wobei die Flexibilität insbesondere durch eine geringe Schichtdicke und/oder eine hohe elastische Verformbarkeit des Kunststoffes erreicht werden kann. Auf die mikrofluidischen Kanäle, die durch die flexible Membranschicht verschlossen sind, wirkt sich die Flexibilität aufgrund der im Vergleich zur Ventilkammer kleineren Angriffsfläche nicht oder nur in äußerst geringem Maße aus.

Die Beaufschlagung der Membran mit Druck kann beispielsweise mechanisch oder hydraulisch erfolgen, bevorzugt aber wird die Membran pneumatisch mit Druck beaufschlagt.

Dadurch, dass der Zuleitungskanal durch den Verbindungskanal mit dem Ableitungskanal verbunden ist und dadurch, dass Ventilkammer und Verbindungskanal so ausgelegt sind, dass die Membran im geschlossenen Zustand zwar den zumindest einen Einleitungskanal nicht aber den Verbindungskanal versperrt, wird erreicht, dass im geschlossenen Zustand der Membran bzw. des Mikrofluid-Ventils trotzdem zuzuführendes Fluid über Zuleitungskanal, Verbindungskanal und Ableitungskanal durch das Mikrofluid-Ventil fließen kann. Mit anderen Worten sind Zuleitungskanal und Ableitungskanal durch den Verbindungskanal kurzgeschlossen bzw. ist die Ventilkammer durch den Verbindungskanal überbrückt, so dass der entsprechende Fluidpfad Mikrofluid-Ventil zwischen Zuleitungskanal und Ableitungskanal auch im geschlossenen Zustand der Membran durchströmbar bleibt.

Durch die erfindungsgemäße Gestaltung lässt sich das Mikrofluid-Ventil in einfacher Art und Weise in ein mikrofluidisches System integrieren: Zuleitungskanal, Verbindungskanal und Ableitungskanal werden als gemeinsamer Durchflusskanal genützt, sodass im geschlossenen Zustand der Membran Fluid ohne eine zusätzliche mikrofluidische Komponenten, wie etwa eine Stichleitung und eine Umgehungsleitung, durch das Mikrofluid-Ventil fließen kann. Somit ist auch der Platzbedarf eines erfindungsgemäßen Mikrofluid-Ventils in einem mikrofluidischen System gegenüber einem herkömmlichen mikrofluidischen Ventil mit zusätzlichen mikrofluidischen Komponenten stark reduziert.

Im geöffneten Zustand der Membran ist der zumindest eine Einleitungskanal freigegeben und somit, je nach Anzahl der Einleitungskanäle, zumindest ein zusätzlicher Fluidpfad freigegeben. Entsprechend kann im geöffneten Zustand der Membran einzuleitendes Fluid aus dem Einleitungskanal bzw. aus einem der Einleitungskanäle über die Ventilkammer in den Ableitungskanal gelangen. In beiden Ventilzuständen kann das durch das Mikrofluid-Ventil geführte Fluid über den Ableitungskanal weitergeführt werden.

Besonders deutlich wird der große Vorteil des erfindungsgemäßen Mikrofluid-Ventils, wenn mehrere Mikrofluid-Ventile seriell miteinander verbunden werden, wobei jeweils der Ableitungskanal des einen Mikrofluid-Ventils mit dem Zuleitugnskanal des nachfolgenden Mikrofluid-Ventils verbunden ist. Der besseren Verständlichkeit halber wird in den nachfolgenden Betrachtungen von lediglich einem Einleitungskanal pro Mikrofluid-Ventil gesprochen, wenngleich wie oben ausgeführt auch mehr als ein Einleitungskanal pro Mikrofluid-Ventil vorgesehen sein kann.

Sind alle Mikrofluid-Ventile geschlossen, kann Fluid über die nicht versperrten Verbindungskanäle ungehindert vom ersten Zuleitungskanal bis zum letzten Ableitungskanal fließen. Ist eines der Mikrofluid-Ventile jedoch geöffnet, kann Fluid aus dem Einleitungskanal des geöffneten Mikrofluid-Ventils durch die Verbindungskanäle der nachgeschalteten geschlossenen Mikrofluid-Ventile zum letzten Ableitungskanal fließen.

Vorteilhaft für die Durchströmung und die Integrierbarkeit des Mikrofluid-Ventils ist es, wenn Zuleitungskanal und Ableitungskanal entlang einer Geraden verlaufend angeordnet sind, insbesondere parallel zur Oberfläche der Trägerschicht. Ebenfalls kann entsprechend vorgesehen sein, dass auch der Verbindungskanal aus Sicht der Membranschicht gesehen - sprich von oben gesehen - auf der Geraden liegt.

Entsprechend lassen sich ein oder mehrere erfindungsgemäße Mikrofluid-Ventile in einfacher Art und Weise in mikrofluidische Systeme, insbesondere in mikrofluidische Chips, integrieren.

In einer Ausführungsvariante der Erfindung ist vorgesehen, dass der Verbindungskanal bezogen auf die flexible Membranschicht unterhalb der Ventilkammer verläuft und in Richtung der Ventilkammer geöffnet ist. Dadurch, dass der Verbindungskanal unterhalb der Ventilkammer verläuft, kann in einfacher Art und Weise sichergestellt werden, dass der Verbindungskanal nicht im geschlossenen Zustand der Membran durch diese verschlossen wird, sondern durchströmbar bleibt. Durch die Öffnung des Verbindungskanals in Richtung der Ventilkammer wird eine fluidische Verbindung zwischen Ventilkammer und Verbindungskanal hergestellt, sodass sichergestellt wird, dass im geöffneten Zustand der Membran Fluid aus dem zumindest einen Einleitungskanal in den Verbindungskanal gelangen kann, sodass das Fluid auch über den Verbindungskanal in den Ableitungskanal weitergeleitet wird. Gleichzeitig kann somit in einfacher Weise sichergestellt werden, dass in der Ventilkammer befindliches Fluid während des Schließens der Membran über den Verbindungskanal aus der Ventilkammer abfließen kann.

Um eine besonders einfache Fertigung des Mikrofluid-Ventils zu ermöglichen, wobei alle mikrofluidischen Kanäle sowie die Ventilkammer und der mikrofluidische Verbindungskanal mit möglichst wenigen Herstellungsschritten, vorzugsweise in einem gemeinsamen Herstellungsschritt, insbesondere durch Einprägen oder lithographische Verfahren, ist gemäß einer weiteren Ausführungsvariante der Erfindung vorgesehen, dass der Verbindungskanal als eine rinnenförmige Vertiefung in der Ventilkammer ausgebildet ist. Der Verbindungskanal erstreckt sich somit von einer Begrenzungsfläche der Ventilkammer in die Trägerschicht. Vorzugsweise ist eine Tiefe der rinnenförmigen Vertiefung bezogen auf die Begrenzungsfläche der Ventilkammer konstant. Der Querschnitt der rinnenförmigen Vertiefung kann mit Ausnahme der durch die Begrenzungsfläche vorgegebenen geometrischen Form beliebig gewählt werden, wobei die rinnenförmige Vertiefung beispielsweise in Form einer Nut ausgeführt sein kann.

Eine weitere Ausführungsvariante der Erfindung sieht vor, dass der Verbindungskanal, vorzugsweise im Bereich der Ventilkammer, bezogen auf die flexible Membranschicht bogenförmig zwischen Zuleitungskanal und Ableitungskanal verlaufend ausgebildet ist. Während Zuleitungskanal, Verbindungskanal und Ableitungskanal aus Richtung der Membranschicht - sprich von oben - betrachtet vorzugsweise geradenförmig verlaufen, um Strömungswiderstände zu reduzieren, verläuft der Verbindungskanal in dieser Ausführungsvariante im Querschnitt - sprich von der Seite - gesehen bogenförmig zwischen Zuleitungskanal und Ableitungskanal. Vorteilhafter Weise ist der Verbindungskanal so gebogen, dass die Bogenform des Verbindungskanals im Bereich der Ventilkammer dem durch die Kugelkalotten-Form der Ventilkammer vorgegebenen Kreisbogen folgt.

Um einen konstanten Durchfluss eines Fluides möglichst ohne lokale Änderungen der Durchflussgeschwindigkeit des Fluides im Bereich des Verbindungskanals zu ermöglichen, wenn die Membran die Ventilkammer verschließt, ist gemäß einer weiteren Ausführungsvariante der Erfindung vorgesehen, dass ein Durchflussquerschnitt des Zuleitungskanals und des Ableitungskanals gleich groß sind und der Verbindungskanal derart dimensioniert ist, dass dessen Durchflussquerschnitt im geschlossenen Zustand der Membran im Wesentlichen konstant ist und dem Durchflussquerschnitt von Zuleitungskanal und Ableitungskanal entspricht.

Die Erfindung betrifft aufgrund der herausragenden Integrierbarkeit der erfindungsgemäßen Mikrofluid-Ventile in ein mikrofluidisches System auch einen mikrofluidischen Chip umfassend eine Chip-Trägerschicht und eine an einer Oberfläche der Chip-Trägerschicht aufgebrachte flexible Chip-Membranschicht, wobei die Chip-Trägerschicht mehrere fluidische Anschlüsse aufweist und ein mit den fluidischen Anschlüssen verbundenes mikrofluidisches Kanalsystem in der Oberfläche der Chip-Trägerschicht ausgebildet ist, wobei mikrofluidische Ventile zur Durchflussregelung des mikrofluidischen Kanalsystems vorgesehen sind. Dabei ist weiters vorgesehen, dass zumindest eines der mikrofluidischen Ventile als erfindungsgemäßes Mikrofluid-Ventil mit jeweils einem Einleitungskanal ausgebildet ist, wobei die flexible Membranschicht des zumindest einen Mikrofluid-Ventils durch die flexible Chip-Membranschicht ausgebildet ist,
wobei die Trägerschicht des zumindest einen Mikrofluid-Ventils durch die Chip-Trägerschicht ausgebildet ist und die mikrofluidischen Kanäle des zumindest einen Mikrofluid-Ventils Teil des mikrofluidischen Kanalsystems sind,
wobei der Einleitungskanal des zumindest einen Mikrofluid-Ventils mit einem der fluidischen Anschlüsse verbunden ist.

Da die Trägerschicht des zumindest einen Mikrofluid-Ventils durch die Chip-Trägerschicht und die flexible Membranschicht des zumindest einen Mikrofluid-Ventils durch die flexible Chip-Membranschicht ausgebildet sind, ist das zumindest eine Mikrofluid-Ventil vollständig in den mikrofluidischen Chip integriert. Mit anderen Worten kann das zumindest eine Mikrofluid-Ventil gemeinsam mit dem mikrofluidischen Chip hergestellt werden, ohne dass zusätzliche Verfahrensschritte oder eine aufwändige Integration in das mikrofluidische Kanalsystem erforderlich wären. Das zumindest eine Mikrofluid-Ventil kann als Zudosierventil verwendet werden, um im geöffneten Zustand der Membran ein Fluid aus dem Einleitungskanal in das mikrofluidische Kanalsystem des Chips einleiten zu können. Im geschlossenen Zustand der Membran kann Fluid über den Verbindungskanal durch das Mikrofluid-Ventil geführt werden, während der Einleitungskanal verschlossen ist.

Ebenfalls lassen sich durch diese Bauart mikrofluidische Ventile mit Kugelkalotten-förmigen Ventilkammern gemäß dem Stand der Technik in einfacher Art und Weise in den mikrofluidischen Chip integrieren, wenn einfache Sperrventile für die Funktion erforderlich sind.

Wie bereits eingangs erwähnt, zeigen sich die besonderen synergistischen Effekt der erfindungsgemäßen Gestaltung der Mikrofluid-Ventile insbesondere dann, wenn mehr als eines der Mikrofluid-Ventile vorgesehen ist und die Mikrofluid-Ventile seriell miteinander verbunden sind. Besonders vorteilhaft kann dies in einem mikrofluidischen Chip eingesetzt werden, der zur Synthese einer Verbindung, vorzugsweise eines Oligonukleotids, in einer Synthesekammer ausgebildet ist und vorzugsweise mehrere Reagenzien zur Durchführung der Synthese der Synthesekammer zugeführt werden müssen. Zum Zwecke des Transports der Reagenzien von den fluidischen Anschlüssen zur Synthesekammer weist der mikrofluidische Chip entsprechend einen Hauptkanal auf. So ist in einer Ausführungsvariante des mikrofluidischen Chips vorgesehen, dass der mikrofluidische Chip eine Synthesekammer zur Synthese eines Oligonukleotids sowie einen mit der Synthesekammer verbundenen Hauptleitungskanal aufweist, wobei eine Mehrzahl der mikrofluidischen Ventile als Mikrofluid-Ventile ausgebildet sind, wobei der Hauptleitungskanal zumindest abschnittsweise durch die Zuleitungskanäle, Ableitungskanäle und Verbindungskanäle der Mikrofluid-Ventile ausgebildet ist. Durch die Integration der Mikrofluid-Ventile in den Hauptkanal selbst, kann Fluid im geschlossenen Zustand der Mikrofluid-Ventile über die entsprechenden Verbindungskanäle ungehindert durch den Hauptkanal strömen. Die Einleitung eines Fluides aus einem fluidischen Anschluss über ein diesem Anschluss zugeordnetes Mikrofluid-Ventil in den Hauptkanal kann in einfacher Art und Weise durch Öffnen des entsprechenden Mikrofluid-Ventils erreicht werden. Die übrigen Mikrofluid-Ventile bleiben dabei geschlossen, um den Durchfluss zu ermöglichen.

Um sicherzustellen, dass die Einleitung bzw. Zudosierung von Reagenzien im mikrofluidischen Chip ausschließlich über Mikrofluid-Ventile erfolgt und ein gemeinsamer Hauptkanal, der jedoch mehrere Äste aufweisen kann, vorgesehen ist, sieht eine weitere Ausführungsvariante vor, dass eine Mehrzahl der fluidischen Anschlüsse als Reagenzien-Anschlüsse zur Zuleitung von Reagenzien aus an die jeweiligen fluidischen Anschlüsse angeschlossenen Reagenzbehältern zur Synthesekammer ausgebildet sind, wobei alle Reagenzien-Anschlüsse über jeweils ein Mikrofluid-Ventil an den Hauptkanal angebunden sind.

Besonders vorteilhaft ist der Einsatz der erfindungsgemäßen Mikrofluid-Ventil-Technologie in mikrofluidischen Chips, die für den Einsatz in einer Vorrichtung zur Synthese von Oligonukleotiden bestimmt ist, wie sie in der WO 2018/153999 beschrieben ist. Dabei erfolgt die Synthese eines Oligonukleotids, vorzugsweise eines DNA Stranges, mit einer vordefinierbaren Sequenzabfolge und Länge mittels der Phosphoramidit-Synthese, wofür eine Mehrzahl an Reagenzien notwendig ist. Entsprechend sieht eine besonders bevorzugte Ausführungsvariante vor, dass der mikrofluidische Chip derart ausgebildet ist, dass dieser in einer automatisierten Vorrichtung zur Synthese von Oligonukleotiden, vorzugweise von DNA-Strängen, mit einer vordefinierbaren Länge und Sequenzabfolge an Hand einer Phosphoramidit-Synthese eingesetzt werden kann, wobei die Ventilstellung der mikrofluidischen Ventile, insbesondere der Mikrofluid-Ventile, des mikrofluidischen Chips mittels der Vorrichtung steuerbar ist.

### KURZE BESCHREIBUNG DER FIGUREN

Die Erfindung wird nun anhand eines Ausführungsbeispiels näher erläutert. Die Zeichnungen sind beispielhaft und sollen den Erfindungsgedanken zwar darlegen, ihn aber keinesfalls einengen oder gar abschließend wiedergeben.

Dabei zeigt:
- Fig. 1: eine isometrische Ansicht eines Mikrofluid-Ventils mit einem Einleitungskanal;
- Fig. 2a: eine schematische Schnittdarstellung des Mikrofluid-Ventils in einem geöffneten Zustand;
- Fig. 2b: eine schematische Schnittdarstellung des Mikrofluid-Ventils in einem geöffneten Zustand;
- Fig. 3: ein Chip-Layout eines mikrofluidischen Chips, in welchen eine Mehrzahl an Mikrofluid-Ventilen integriert ist.

### WEGE ZUR AUSFÜHRUNG DER ERFINDUNG

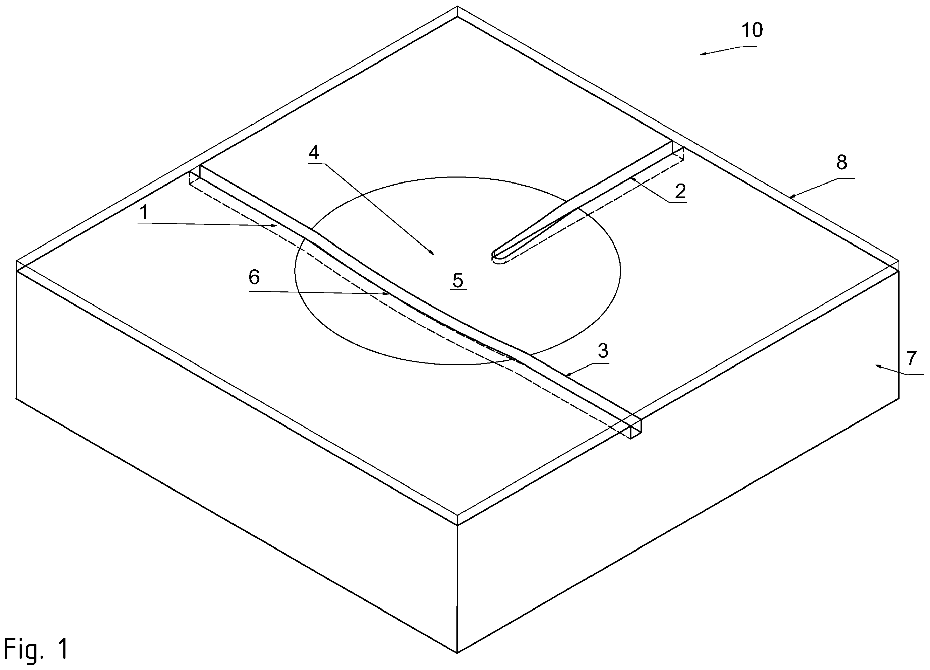

Fig. 1 zeigt eine vergrößerte schematische Darstellung eines Ausführungsbeispiels eines erfindungsgemäßen Mikrofluid-Ventils 10 in einer isometrischen Ansicht. Das Mikrofluid-Ventil 10 besteht aus einer Trägerschicht 7 und einer unmittelbar auf der Trägerschicht 7 aufgebrachten flexiblen Membranschicht 8, welche Membranschicht 8 eine Oberseite der Trägerschicht 7, im gegenständlichen Ausführungsbeispiel, vollständig bedeckt.

In einer die Oberseite ausbildenden Oberfläche der Trägerschicht 7 sind mehrere mikrofluidische Kanäle 1,2,3,6 sowie eine Ventilkammer 5 in Form einer Kugelkalotte ausgebildet. Diese Strukturen können beispielsweise in die Trägerschicht 7 eingeprägt werden oder mittels eines lithographischen Verfahrens hergestellt werden.

Im Bereich der Ventilkammer 5 bildet die flexible Membranschicht 8 eine flexible Membran 4 aus, welche durch Beaufschlagung mit Druck in die Ventilkammer 5 gepresst werden kann.

Die mikrofluidischen Kanäle 1,2,3,6 umfassen einen Zuleitungskanal 1 und einen Ableitungskanal 3 sowie einen Einleitungskanal 2, welche allesamt in die Ventilkammer 5 einmünden.

Betrachtet man das Mikrofluid-Ventil 10 von oben, sprich entlang einer Vertikalachse, sodass der Umriss der Ventilkammer 5 kreisförmig erscheint, so sind im vorliegenden Ausführungsbeispiel Zuleitungskanal 1 und Ableitungskanal 3 entlang einer Geraden verlaufend angeordnet, wobei die Gerade als Sekante durch den kreisförmigen Umriss der Ventilkammer 5 verläuft. Der Einlasskanal 2 verläuft in dieser Ansicht radial in Richtung des gedachten Mittelpunkts des kreisförmigen Umrisses der Ventilkammer 5 und quer zur durch Zuleitungskanal 1 und Ableitungskanal 3 definierten Gerade. Im vorliegenden Ausführungsbeispiel schließt die durch Zuleitungskanal 1 und Ableitungskanal 3 definierten Gerade einen rechten Winkel mit dem Einleitungskanal 2 ein, wobei der Einleitungskanal 2 nicht in dem durch die Sekante abgetrennten Kreissegment angeordnet ist.

Zusätzlich ist ein mikrofluidischer Verbindungskanal 6 vorgesehen, der den Zuleitungskanal 1 und den Ableitungskanal 3 miteinander verbindet, wobei der Verbindungskanal 6 als rinnenförmige Vertiefung in der Ventilkammer 5 ausgebildet ist, die von oben gesehen als Sekante durch die Ventilkammer 5 verläuft. Im vorliegenden Ausführungsbeispiel liegt der Verbindungskanal auf derselben Geraden wie Zuleitungskanal 1 und Ableitungskanal 3.

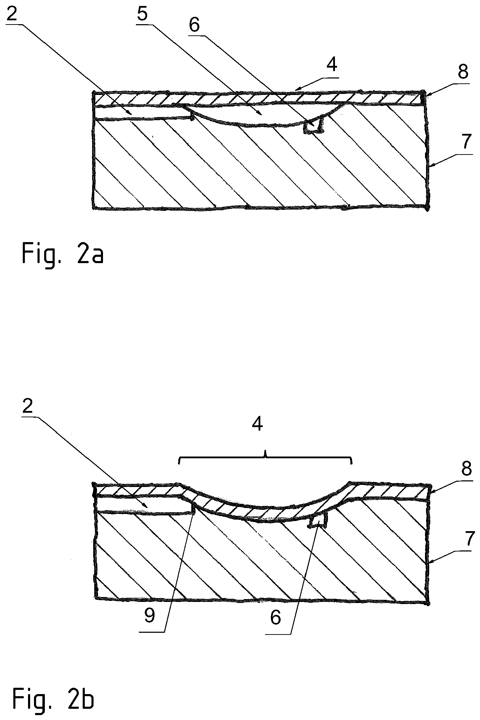

Die Funktionsweise des Mikrofluid-Ventils 10 wird aus den Figuren 2a und 2b deutlich. In Fig. 2a ist die Membran 4 entspannt und die Ventilkammer 5 somit freigegeben. Somit sind grundsätzlich drei Fluidpfade möglich: erstens kann Fluid aus dem Zuleitungskanal 1 über die freigegebene Ventilkammer 5 in den Ableitungskanal 3 gelangen, zweitens kann Fluid aus dem Einleitungskanal 2 über die Ventilkammer 5 in den Ableitungskanal 3 gelangen und drittens können auch gleichzeitig Fluide aus Zuleitungskanal 1 und Einleitungskanal 2 in die Ventilkammer 5 gelangen und gemeinsam über den Ableitungskanal 3 abfließen.

Der Einleitungskanal 2 erstreckt sich in radialer Richtung in die Ventilkammer 5 hinein, wobei eine Kanaltiefe (in Vertikalrichtung von der entspannten Membranschicht 8 aus gemessen) des Einleitungskanals 2 größer ist als eine Tiefe der Ventilkammer 5 an jedem Punkt, an dem der Einleitungskanal 2 in radialer Richtung in der Ventilkammer 5 endet. Somit bildet sich eine Dichtkante 9 aus und es wird erreicht, dass in der Ventilkammer 5 befindliches Fluid, welches aus dem Einleitungskanal 2 stammt, beim Schließen der Membran 4 in den Einleitungskanal 2 zurückgedrängt werden kann bzw. beim Öffnen in einfacher Weise in die Ventilkammer 5 einströmen kann.

Wie ebenfalls ersichtlich ist, verläuft der Verbindungskanal 6 bezogen auf die Membranschicht 8 unterhalb der Ventilkammer 5 und ist in Richtung der Ventilkammer 5 geöffnet.

Diese Gestaltung von Ventilkammer 5, Einleitungskanal 2 und Verbindungskanal 6 ermöglicht es, dass die Membran 4 - wie in Fig. 2b zu sehen ist - durch Beaufschlagung mit Druck in einen geschlossenen Zustand bringbar ist, in welchem geschlossenen Zustand die Membran 4 in die Ventilkammer 5 gepresst wird, um den Übertritt eines einzuleitenden Fluides aus dem Einleitungskanal 2 in die Ventilkammer 5 zu verhindern während ein zuzuführendes Fluid aus dem Zuleitungskanal 1 über den Verbindungskanal 6 in den Ableitungskanal 3 fließen kann, obwohl die Ventilkammer 5 durch die Membran 4 verschlossen bzw. ausgefüllt ist. Die Membran 4 bildet dabei auch eine obere Begrenzung des in Richtung der Ventilkammer 5 geöffneten Verbindungskanals 6 aus.

Mit anderen Worten ist es mit dem diskutierten Mikrofluid-Ventil 10 möglich, dass im geschlossenen Zustand der Membran 4 die Ventilkammer 5 durch den Verbindungskanal 6 überbrückt ist, um einen Fluidtransport von Zuleitungskanal 1 zum Ableitungskanal 3 zu ermöglichen. Entsprechend ist im geschlossenen Zustand der Membran 4 der Fluidpfad zwischen Zuleitungskanal 1 und Ableitungskanal 3 über den Verbindungskanal 6 offen.

Wie ebenfalls zu sehen ist, wird die Membran 4 im geschlossenen Zustand an die Begrenzungsfläche der Ventilkammer 5 gepresst und verschließt so einerseits den Einleitungskanal 2, vorzugsweise über die umlaufende Dichtkante 9, und verhindert andererseits den direkten Übertritt von Fluid aus Zuleitungskanal 1 oder Ableitungskanal 3 in die (verschlossene) Ventilkammer 5.

In einem (nicht dargestellten) Schnitt durch Zuleitungskanal 1, Verbindungskanal 6 und Ableitungskanal 3 parallel zur Vertikalrichtung verläuft der Verbindungskanal 6 bogenförmig zwischen Zuleitungskanal 1 und Ableitungskanal 3.

Die Abmessungen bzw. die geometrische Ausgestaltung des Verbindungskanals 6 ist vorzugsweise so gewählt, dass ein Durchflussquerschnitt von Zuleitungskanal 1, Verbindungskanal 6 und Ableitungskanal 3 im geschlossenen Zustand der Membran 4, sprich wenn die Membran 4 den Verbindungskanal 6 nach oben hin verschließt, im Wesentlichen konstant ist.

Wenngleich im zuvor beschriebenen Ausführungsbeispiel lediglich ein Einleitungskanal 2 vorgesehen ist, ist es auch denkbar zwei oder mehrere in die Ventilkammer 5 mündende Einleitungskanäle 2 vorzusehen, um zusätzliche Fluidpfade zu schaffen.

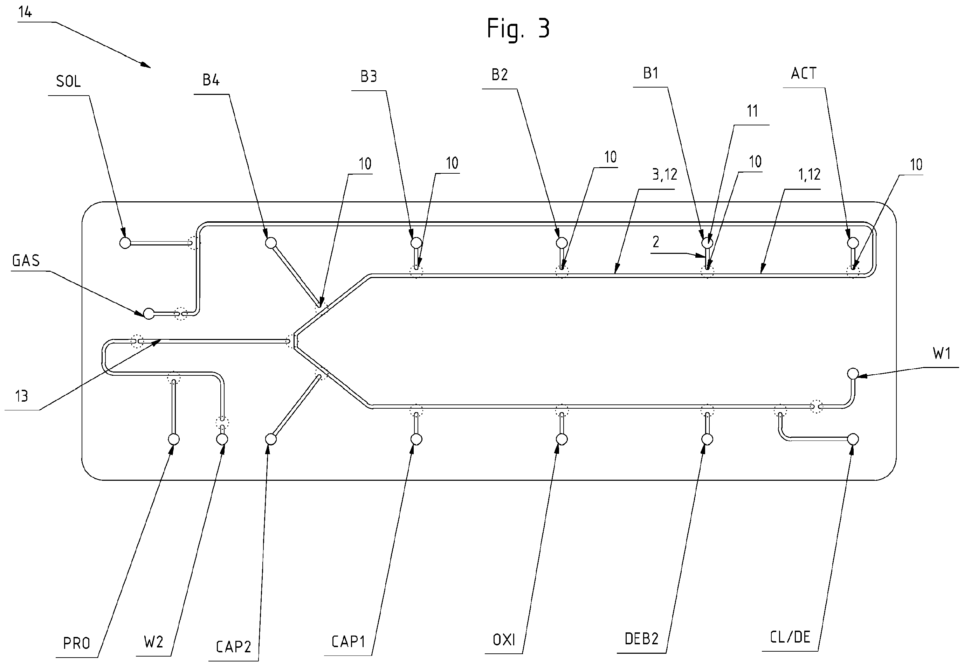

Figur 3 zeigt ein mögliches Layout eines mikrofluidischen Chips 14, mit dem eine Synthese von Oligonukleotiden in einer automatisierten Vorrichtung möglich ist, wobei mehrere der mikrofluidischen Ventile des mikrofluidischen Chips 14 als Mikrofluid-Ventile 10 ausgebildet sind.

Der mikrofluidische Chip 14 weist eine Vielzahl von fluidischen Anschlüssen 11 auf, wobei jene fluidischen Anschlüsse 11, die für die Zufuhr eines für die Synthese notwendigen Reagenz über ein Mikrofluid-Ventil 10 an einen Hauptleitungskanal 12 des mikrofluidischen Chips 14 angebunden sind. Zusätzlich können auch herkömmliche mikrofluidische Ventile vorgesehen sein, wobei der Hauptleitungskanal 12 mit einer Synthesekammer 13 verbunden ist. In der Synthesekammer 14 befindet sich ein Trägermedium mit Linker-Molekülen, welche als Ausgangspunkt für die Synthese der Oligonukleotide fungieren. Die Mikrofluid-Ventile 10, sowie vorzugsweise auch die übrigen mikrofluidischen Ventile, sind vollständig in den mikrofluidischen Chip 14 integriert und einstückig ausgebildet. Dies wird erreicht, indem die Ventilkammern 5 und die mikrofluidischen Kanäle 1,2,3,6 der Mikrofluid-Ventile 10 in einer Chip-Trägerschicht ausgebildet sind und somit die Trägerschicht 7 durch die Chip-Trägerschicht ausgebildet ist. Auch die flexible Membranschicht 8 der Mikrofluid-Ventile 10 wird durch eine Chip-Membranschicht ausgebildet.

Wie die Durchströmung der fluidischen Verbindung 13 des mikrofluidischen Chips 10 bewerkstelligt wird, wird in der Folge anhand der ersten Base B1 beschrieben. Dieses Prinzip lässt sich analog auf alle anderen Reagenzien anwenden.

Der fluidische Anschluss 11 der ersten Base B1 ist mit einem Einleitungskanal 2 des entsprechenden Mikrofluid-Ventils 10 verbunden. Der Zuleitungskanal 1, der Verbindungskanal 6 und der Ableitungskanal 3 sind als Teil des Hauptleitungskanals 12 des Chips 14 ausgebildet. Ist das der ersten Base B1 zugeordnete Mikrofluid-Ventil 10 geschlossen, so kann Fluid aus weiter von der Synthesekammer 13 entfernten fluidischen Anschlüssen 11, beispielsweise Lösungsmittel SOL oder Reagenz zur Aktivierung einer detritylierten 5'-OH-Gruppe ACT - bei entsprechendem geöffneten Mikrofluid-Ventil 10, durch das der ersten Base B1 zugeordnete Mikrofluid-Ventil 10 bzw. durch den von diesem Mikrofluid-Ventil 10 gebildeten Abschnitt des Hauptleitungskanals 12 fließen, ohne dass sich dieses Fluid mit der ersten Base B1 vermischt.

Ist das der ersten Base B1 zugeordnete Mikrofluid-Ventil 10 geöffnet, so kann die erste Base B1 über den Einleitungskanal 2 und die Ventilkammer 5 des Mikrofluid-Ventils 10 in den Hauptleitungskanal 12 gelangen und durströmt in der Folge die nachgeschalteten, geschlossenen Mikrofluid-Ventile 10.

Um den Durchfluss zu ermöglichen und entsprechende Druckverhältnisse herzustellen, wird zusätzlich ein mikrofluidisches Ventil geöffnet, welches einen als zweiten Auslass W2 ausgebildeten fluidischen Auslassanschluss steuert. Der zweite Auslass W2 ist in Strömungsrichtung hinter der Synthesekammer 13 angeordnet, sodass die erste Base B1 über den Hauptleitungskanal 12 und die Synthesekammer 13 zum zweiten Auslass W2 strömt. Der zweite Auslass W2 kann beispielsweise mit einem Abfallbehälter verbunden sein.

Die mikrofluidischen Ventile und die Mikrofluid-Ventile 10 sind grundsätzlich in einer geschlossenen Stellung gehalten, sodass die den Reagenzien zugeordneten Einleitungskanäle 2 versperrt sind und lediglich der Hauptleitungskanal 12 geöffnet ist. Die geschlossene Stellung wird beispielsweise dadurch erreicht, dass eine Steuereinrichtung einer automatisierten und programmierbaren Synthesevorrichtung über ein pneumatisches System, etwa über Steuerleitungen 28, Druck auf die Membranen 4 der Mikrofluid-Ventile 10 bzw. auf die mikrofluidischen Ventile ausübt. Wird von der Steuereinrichtung der auf eines der mikrofluidischen Ventile bzw. Mikrofluid-Ventile 10 ausgeübte Druck verringert bzw. wird die Druckbeaufschlagung ausgesetzt, so öffnet sich das entsprechende mikrofluidische Ventil bzw. Mikrofluid-Ventil 10 und die fluidische Verbindung zwischen dem entsprechenden Einleitungskanal 2 und dem Hauptleitungskanal 12 ist hergestellt bzw. offen.

Bevor die Synthese starten kann, ist es notwendig, die Reagenzien aus Reagenzbehältern über Transportleitungen an die Mikrofluid-Ventile 10 heranzuführen. Daher werden der Reihe nach die Mikrofluid-Ventile 10 für die fluidischen Anschlüsse 11 der für die Synthese notwendigen Reagenzien jeweils einzlen gemeinsam mit dem mikrofluidischen Ventil für einen ersten Auslass W1 geöffnet. Zwischen den einzelnen Heranführungen wird der Hauptleitungskanal 12 jeweils durch gleichzeitiges Öffnen des Mikrofluid-Ventils 10 für das Lösungsmittel SOL und des mikrofluidischen Ventils für den ersten Auslass W1 zuerst gespült und danach durch Öffnen des mikrofluidischen Ventils für das Inertgas GAS und des mikrofluidischen Ventils für den ersten Auslass W1 getrocknet. Dadurch gelangt beim Heranführen, beim Spülen und beim Trocknen keine der Reagenzien in die Synthesekammer 13. Durch gleichzeitiges Öffnen des mikrofluidischen Ventils für das Inertgas GAS und jeweils einem der Mikrofluid-Ventile 10 für die fluidischen Anschlüsse 11 der für die Synthese notwendigen Reagenzien, können die Reagenzien nach dem Ende der Synthese wieder zurück in die Reagenzbehälter befördert werden.

In der Folge sollen nun die Syntheseschritte eines Synthesezyklus besprochen werden, die zur Koppelung eines Nukleotids an das Ende einer Teilsequenz eines Oligonukleotids bzw. als erstes Nukleotid an ein Linker-Molekül eines Trägermediums notwendig sind. Die Abfolge der Syntheseschritte und der dafür verwendeten Reagenzien ist an sich bekannt.

Jedes Oligonukleotid startet an einem Linker-Molekül eines in der Synthesekammer 13 angeordneten Trägermediums und wird mit jedem Syntheseschritt um ein Nukleotid erweitert, welches an das Ende der Kette gekoppelt wird. Die 5'-OH-Gruppe des Oligonukleotids ist mit einer säurelabilen Dimethoxytrityl-Schutzgruppe (4,4'-Dimethoxytrityl - DMT) versehen.

Zuerst wird der Synthesekammer 13 ein Reagenz zur Detritylierung DEBL eines Endes eines eine Teilsequenz aufweisenden Oligonukleotids bzw. zur Detrytilierung des Linker-Moleküls aus dem entsprechenden Reagenzbehälter zugeführt. Dabei wird wie zuvor beschrieben das den fluidischen Anschluss 11 für das Reagenz zur Detritylierung DEBL steuernde Mikrofluid-Ventil 10 gemeinsam mit dem den zweiten Auslass W2 steuernden mikrofluidischen Ventil geöffnet.

Dadurch wird die DMT-Schutzgruppe entfernt, sodass ein weiteres Nukleotid an die freie 5'-OH-Gruppe angekoppelt werden kann. Bei dem Reagenz zur Detritylierung DEBL handelt es sich im vorliegenden Fall um eine saure Lösung, nämlich eine Lösung enthaltend 2% Trichloressigsäure oder 3% Dichloressigsäure in einem inerten Lösungsmittel wie Dichlormethan oder Toluol. Dieser Schritt wird auch als Deblocking-Schritt bezeichnet.

Im nächsten Schritt wird die Nukleotidkette an der detritylierten, freien 5'-OH-Gruppe um eine Base, also entweder Adenin B1, Guanin B2, Cytosin B3 oder Thymin B4 für einen DNA Strang oder Uracil B4 für einen RNA Strang, verlängert. Dazu wird der Synthesekammer 14 abwechselnd ein Reagenz zur Aktivierung ACT der freien 5'-OH-Gruppe und ein Reagenz enthaltend Phosphoramidit der entsprechenden Base B1,B2,B3,B4 zugeführt. Die Phosporamidite werden in einem Lösungsmittel SOL, insbesondere in Acetonitril, gelöst zugeführt. Die Aktivierung der 5'-OH-Gruppe wird im vorliegenden Fall mittels einer 0,2-0,7 molaren Lösung eines sauren Azol-Katalysators erreicht, insbesondere durch 1H-Tetrazol, 5-Ethylthio-1H-Tetrazol, 2-Benzylthiotetrazol oder 4,5-Dicyanoimidazol. Dabei koppelt das Nukleotid an die freie 5'-OH-Gruppe des Oligonukleotids, während der Phosphoramidit-Rest abgespalten wird. Die 5'-OH-Gruppe des neu gekoppelten Nukleotids ist wiederum durch eine DMT-Schutzgruppe geschützt. Dieser Schritt wird auch als Coupling-Schritt bezeichnet.

In der Synthesekammer 13 wird im nächsten Schritt eine Mischung aus zwei Reagenzien zur Blockierung CAP1,CAP2 nicht umgesetzter 5'-OH-Gruppen zugeführt. Im vorliegenden Fall wird die Blockierung nicht umgesetzter 5'-OH-Gruppen durch eine Mischung aus Acetanhydrid und 1-Methylimidazol als Katalysator erreicht. Dieser Schritt wird auch als Capping-Schritt bezeichnet.

Als letzter Schritt eines Synthesezyklus erfolgt die Oxidation einer Phosphittriester-Bindung, die zwischen dem neu gekoppelten Nukleotid und der entsprechenden 5'-OH-Gruppe des Oligonukleotids ausgebildet ist, indem ein Reagenz zur Oxidation OXI zugeführt wird. Das Reagenz zur Oxidierung OXI oxidiert die Phosphittriester-Bindung in einen vierfach koordinierten Phosphotriester, einen geschützten Vorläufer der natürlich vorkommenden Phosphatdiester-Internukleosidbindung. Dadurch wird die Bindung zwischen dem angekoppelten Nukleotid und der entsprechenden 5'-OH-Gruppe stabilisiert. Die Oxidation wird im vorliegenden Fall unter wasserfreien Bedingungen mittels (1S)-(+)-(10-Camphersulfonyl)-oxaziridin (CSO) erreicht. Dieser Schritt wird als Oxidations-Schritt bezeichnet.

Die vier Schritte eines Synthesezyklus werden in der Reihenfolge der Nukleotidsequenz des zu synthetisierenden Oligonukleotids so oft wiederholt, bis das Oligonukleotid die vorbestimmte Länge und Sequenz aufweist. Die Reihenfolge der Nukleotidsequenz kann beispielsweise durch eine entsprechend automatisierte Vorrichtung vorgegeben und von einem Nutzer einstellbar sein, sodass eine Vielzahl von unterschiedlichen individuell vorgebbaren Oligonukleotiden in einer Vorrichtung mit einem derartigten Chip 14 hegestellt werden kann.

Sobald das zu synthetisierende Oligonukleotid fertiggestellt ist, wird der Synthesekammer 13 ein Reagenz zur Abspaltung der Oligonukleotide von den Linker-Molekülen und zur Entfernung der Schutzgruppen CL/DE zugeführt. Im vorliegenden Fall wird als Reagenz zur Abspaltung der Oligonukleotide von den Linker-Molekülen und zur Entfernung der Schutzgruppen CL/DE eine Mischung aus Ammoniak und Methylamin eingesetzt, wobei die beide Reagenzien vorzugsweise zu gleichen Teilen in der Mischung enthalten sind. Dieses Reagenz CL/DE löst die Oligonukleotide von den Linker-Molekülen, wobei dieser Vorgang etwa 3 bis 15 Minuten, in der Regel etwa 5 Minuten, dauert. Dabei ist das den fluidischen Anschluss 11 für einen Produktsammelbehälter PRO steuernde mikrofluidische Ventil geöffnet, um die synthetisierten Oligonukleotide in den Produktsammelbehälter PRO zu fördern. Das Entfernen der Schutzgruppen dauert in der Regel weitere 3 bis 15 Minuten, in der Regel etwa 5 Minuten, wenn der Produktsammelbehälter PRO, beispielsweise über einen eingebauten Heizblock, auf eine Temperatur zwischen 50° und 75°, vorzugsweise etwa 65°C, gebracht wird. Bei Raumtemperatur benötigt dieser Vorgang zwischen 45 und 120 Minuten, in der Regel etwa 60 Minuten.

Nach der Beendigung der Synthese kann der Produktsammelbehälter PRO entnommen und weiter verarbeitet werden.

Es versteht sich von selbst, dass die beschriebenen Mikrofluid-Ventile 10 in einer Vielzahl von unterschiedlichen mikrofluidischen Systemen und mikrofluidischen Chips eingesetzt werden können.

### BEZUGSZEICHENLISTE

- 1: Zuleitungskanal
- 2: Einleitungskanal
- 3: Ableitungskanal
- 4: Membran
- 5: Ventilkammer
- 6: Verbindungskanal
- 7: Trägerschicht
- 8: flexible Membranschicht
- 9: Dichtkante
- 10: Mikrofluid-Ventil
- 11: fluidischer Anschluss
- 12: Hauptleitungskanal
- 13: Synthesekammer
- 14: mikrofluidischer Chip

- SOL: Lösungsmittel
- GAS: Inertgas
- ACT: Reagenz zur Aktivierung einer detritylierten 5'-OH-Gruppe
- B1: Base 1 (Bspw. Phosphoramidit der Base Adenin)
- B2: Base 2 (Bspw. Phosphoramidit der Base Guanin)
- B3: Base 3 (Bspw. Phosphoramidit der Base Cytosin)
- B4: Base 4 (Bspw. Phosphoramidit der Base Thymin oder Uracil)
- W1: erster Auslass
- CL/DE: Reagenz zur Abspaltung der Oligonukleotide von den Linker-Molekülen und/oder einer Reagenz zur Entfernung der Schutzgruppen
- DEBL: Reagenz zur Detritylierung einer mit einer Dimethoxytrityl-Schutzgruppe versehenen 5'-OH-Gruppe
- OXI: Reagenz zur Oxidation einer Phosphittriesterbindung
- CAP1: erstes Reagenz zur Blockierung nicht umgesetzter 5'-OH-Gruppen
- CAP2: zweites Reagenz zur Blockierung nicht umgesetzter 5'-OH-Gruppen
- PRO: Produktsammelbehälter
- W2: zweiter Auslass

## Patentansprüche

1. Mikrofluid-Ventil (10) umfassend eine Trägerschicht (7) und eine auf einer Oberfläche der Trägerschicht (7) angeordnete flexible Membranschicht (8),
wobei die Oberfläche der Trägerschicht (7) eine Ventilkammer (3) in Form einer Kugelkalotte aufweist und eine durch die flexible Membranschicht (8) ausgebildete Membran (4) zumindest die Ventilkammer (5) überspannt, wobei mehrere in die Ventilkammer (5) einmündende mikrofluidische Kanäle (1,2,3) in der Oberfläche der Trägerschicht (7) ausgebildet sind,
**dadurch gekennzeichnet, dass**
die mikrofluidischen Kanäle (1,2,3) einen Zuleitungskanal (1), einen Ableitungskanal (3) und zumindest einen Einleitungskanal (2) umfassen,
wobei die mikrofluidischen Kanäle (1,2,3) und die Membran (4) derart ausgebildet sind, dass die Membran (4) durch Beaufschlagung mit Druck in einen geschlossenen Zustand bringbar ist, in welchem geschlossenen Zustand die Membran (4) in die Ventilkammer (5) gepresst wird, um den Übertritt eines einzuleitenden Fluides aus dem zumindest einen Einleitungskanal (2) in die Ventilkammer (5) zu verhindern,
wobei der Zuleitungskanal (1) und der Ableitungskanal (3) durch einen mikrofluidischen Verbindungskanal (6) miteinander verbunden sind,
wobei der Verbindungskanal (6) und die Ventilkammer (5) derart zueinander positioniert sind,
dass im geschlossenen Zustand der Membran (4) ein zuzuführendes Fluid aus dem Zuleitungskanal (1) über den Verbindungskanal (6) in den Ableitungskanal (3) fließen kann, während der zumindest eine Einleitungskanal (2) durch die Membran (4) verschlossen ist
und dass in einem geöffneten Zustand der Membran (4) ein zuzuführendes Fluid aus dem Zuleitungskanal (1) und/oder zumindest ein einzuleitendes Fluid aus dem zumindest einen Einleitungskanal (2) in die Ventilkammer (5) fließen können, wobei ein sich in der Ventilkammer (5) befindliches Fluid über den Ableitungskanal (3) aus der Ventilkammer (5) abfließen kann.

2. Mikrofluid-Ventil (10) nach Anspruch 1, **dadurch gekennzeichnet, dass** der Verbindungskanal (6) bezogen auf die flexible Membranschicht (8) unterhalb der Ventilkammer (5) verläuft und in Richtung der Ventilkammer (5) geöffnet ist.

3. Mikrofluid-Ventil (10) nach einem der Ansprüche 1 bis 2, **dadurch gekennzeichnet, dass** der Verbindungskanal (6) als eine rinnenförmige Vertiefung in der Ventilkammer (5) ausgebildet ist.

4. Mikrofluid-Ventil (10) nach einem der Ansprüche 1 bis 3, **dadurch gekennzeichnet, dass** der Verbindungskanal (6), vorzugsweise im Bereich der Ventilkammer (5), bezogen auf die die flexible Membranschicht (8) bogenförmig zwischen Zuleitungskanal (1) und Ableitungskanal (3) verlaufend ausgebildet ist.

5. Mikrofluid-Ventil (10) nach einem der Ansprüche 1 bis 4, **dadurch gekennzeichnet, dass** ein Durchflussquerschnitt des Zuleitungskanals (1) und des Ableitungskanals (3) gleich groß sind und der Verbindungskanal (6) derart dimensioniert ist, dass dessen Durchflussquerschnitt im geschlossenen Zustand der Membran (4) im Wesentlichen konstant ist und dem Durchflussquerschnitt von Zuleitungskanal (1) und Ableitungskanals (3) entspricht.

6. Mikrofluidischer Chip (14) umfassend eine Chip-Trägerschicht und eine an einer Oberfläche der Chip-Trägerschicht aufgebrachte flexible Chip-Membranschicht, wobei die Chip-Trägerschicht mehrere fluidische Anschlüsse (11) aufweist und ein mit den fluidischen Anschlüssen verbundenes mikrofluidisches Kanalsystem in der Oberfläche der Chip-Trägerschicht ausgebildet ist, wobei mikrofluidische Ventile zur Durchflussregelung des mikrofluidischen Kanalsystems vorgesehen sind, **dadurch gekennzeichnet, dass** zumindest eines der mikrofluidischen Ventile als Mikrofluid-Ventil (10) nach einem der Ansprüche 1 bis 5 mit jeweils einem Einleitungskanal (2) ausgebildet ist,
wobei die flexible Membranschicht (8) des zumindest einen Mikrofluid-Ventils (10) durch die flexible Chip-Membranschicht ausgebildet ist,
wobei die Trägerschicht (7) des zumindest einen Mikrofluid-Ventils (10) durch die Chip-Trägerschicht ausgebildet ist und die mikrofluidischen Kanäle (1,2,3) des zumindest einen Mikrofluid-Ventils (10) Teil des mikrofluidischen Kanalsystems sind,
wobei der Einleitungskanal (2) des zumindest einen Mikrofluid-Ventils (10) mit einem der fluidischen Anschlüsse (11) verbunden ist.

7. Mikrofluidischer Chip (14) nach Anspruch 6, **dadurch gekennzeichnet, dass** der mikrofluidische Chip eine Synthesekammer (13) zur Synthese eines Oligonukleotids sowie einen mit der Synthesekammer (13) verbundenen Hauptleitungskanal (12) aufweist,
wobei eine Mehrzahl der mikrofluidischen Ventile als Mikrofluid-Ventile (10) ausgebildet sind, wobei der Hauptleitungskanal (12) zumindest abschnittsweise durch die Zuleitungskanäle (1), Ableitungskanäle (3) und Verbindungskanäle (2) der Mikrofluid-Ventile (10) ausgebildet ist.

8. Mikrofluidischer Chip (14) nach Anspruch 7, **dadurch gekennzeichnet, dass** eine Mehrzahl der fluidischen Anschlüsse (11) als Reagenzien-Anschlüsse zur Zuleitung von Reagenzien aus an die jeweiligen fluidischen Anschlüsse (11) angeschlossenen Reagenzbehältern zur Synthesekammer (13) ausgebildet sind, wobei alle Reagenzien-Anschlüsse über jeweils ein Mikrofluid-Ventil (10) an den Hauptkanal (12) angebunden sind.

9. Mikrofluidischer Chip (14) nach einem der Ansprüche 6 bis 8, **dadurch gekennzeichnet, dass** der mikrofluidische Chip (14) derart ausgebildet ist, dass dieser in einer automatisierten Vorrichtung zur Synthese von Oligonukleotiden, vorzugweise von DNA-Strängen, mit einer vordefinierbaren Länge und Sequenz an Hand einer Phosphoramidit-Synthese eingesetzt werden kann, wobei die Ventilstellung der mikrofluidischen Ventile, insbesondere der Mikrofluid-Ventile (10), des mikrofluidischen Chips (14) mittels der Vorrichtung steuerbar ist.
